# EUROPEAN PATENT SPECIFICATION

(11) **EP 4 162 866 B1**
(45) Date of publication and mention of the grant of the patent: **11.11.2026**
(21) Application number: 22200253.7
(22) Date of filing: 07.10.2022
(51) Int. Cl.: A61B 5/00, A61B 5/0538

(54) **MEASURING TISSUE PROXIMITY FOR MULTI-ELECTRODE CATHETER**
MESSUNG DER GEWEBENÄHE FÜR KATHETER MIT MEHREREN ELEKTRODEN
MESURE DE LA PROXIMITÉ TISSULAIRE POUR CATHÉTER À ÉLECTRODES MULTIPLES

(30) Priority: 08.10.2021 US 202117496976
(43) Date of publication of application: 12.04.2023
(73) Proprietor: Biosense Webster (Israel) Ltd., 2066717 Yokneam (IL)
(72) Inventor: GOVARI, Assaf, 2066717 Yokneam (IL); ALTMANN, Andres Claudio, 2066717 Yokneam (IL); GLINER, Vadim, 2066717 Yokneam (IL); BOUMENDIL, Alon, 2066717 Yokneam (IL); AVNI, Uri, 2066717 Yokneam (IL)
(74) Representative: Carpmaels & Ransford LLP

(56) References cited:
- EP-A1- 3 473 177
- WO-A1-2016/134264
- WO-A2-2014/036439
- US-A1- 2019 183 378

## Description

### FIELD OF THE INVENTION

The present invention relates generally to medical probes, and particularly to multi-electrode catheters.

### BACKGROUND OF THE INVENTION

Various techniques for verifying contact of an electrode of a catheter with cardiac tissue have been proposed in the patent literature. For example, U.S. Patent Application Publication 2007/0255162 describes methods and systems for providing tissue contact assessment by providing a catheter having a shaft having a plurality of electrodes, positioning the catheter at a tissue treatment site, applying an electrical current between at least two of the plurality of electrodes, measuring impedance voltage between the at least two of the plurality of electrodes and, processing the measured impedance voltage caused by the applied electrical current to provide contact assessment.

As another example, U.S. Patent Application Publication 2020/0038101 describes an apparatus including a shaft and an end effector at a distal end of the shaft. The end effector has a distal end and a proximal end with a longitudinal intermediate point between the distal and proximal ends. The end effector is sized to fit in an anatomical passageway within a cardiovascular system. The end effector includes at least one sensor electrode and a reference electrode. The at least one sensor electrode is configured to contact cardiovascular tissue and thereby pick up potentials. The reference electrode is configured to pick up a potential from fluid in contact with the reference electrode. The reference electrode is located proximal to the longitudinal intermediate point of the end effector. The end effector is configured to prevent the reference electrode from contacting tissue.

An electrophysiological use of an electrode disposed in an interior of a basket catheter was previously reported. For example, U.S. Patent 10,045,707 describes cardiac catheterization performed with a catheter having a basket-shaped assembly at its distal end. A plurality of spline electrodes is disposed on the splines of the assembly. A far-field electrode is disposed in the interior of the assembly. An intracardiac electrogram and a far-field electrogram are obtained with at least one of the spline electrodes and the far-field electrode, respectively. The far-field component is removed from the intracardiac electrogram using the far-field electrogram.

US 2019/183378 A1 relates to a system and method for measuring impedance across a plurality of electrodes and assessing proximity or contact between electrodes of a medical device and patient tissue.

WO 2016/134264 A1 relates to devices and methods for making and using medical devices, including a system for sensing tissue contact.

EP 3473177 A1 relates to A processor that is configured to construct a baseline impedance model (BIM) that models a portion of a heart of a subject as a collection of three-dimensional cells.

### SUMMARY OF THE INVENTION

The invention is defined in claim 1. Embodiments of the invention are defined in the dependent claims.

The present invention will be more fully understood from the following detailed description of the embodiments thereof, taken together with the drawings in which:

### BRIEF DESCRIPTION OF THE DRAWINGS

Fig. 1 is a schematic pictorial illustration of a catheter-based electrophysiological (EP) mapping, positiontracking and ablation system comprising a basket catheter, in accordance with an embodiment of the present invention;
Fig.2 is a perspective, schematic view of the basket catheter of Fig. 1 in physical contact with cavity wall tissue, in accordance with an embodiment of the present invention;
Figs. 3A and 3B are schematic electrical diagrams of a functional electrode electrically coupled to an interior electrode while the functional electrode is in a blood pool and in contact with wall tissue, respectively, in accordance with an embodiment of the present invention; and
Fig. 4 is a flow chart that schematically illustrates a method and algorithm for determining functional electrode contact with wall tissue using the interior electrode of Fig. 2, in accordance with an embodiment of the present invention.

### DETAILED DESCRIPTION OF EMBODIMENTS

### OVERVIEW

A multi-electrode cardiac catheter typically comprises a distal-end assembly onto which multiple electrodes are disposed. For example, a basket catheter typically comprises an expandable frame of splines as a type of a distal-end assembly, that is coupled to the distal end of a shaft for insertion into a cavity of an organ of a patient. As another example, a catheter with multi-arm distal end assembly has multiple arms that open, which carry the electrodes. Alternatively, a multi-electrode catheter may be made from a distal end configured to capture a preformed shape, such as an arcuate shape of a lasso.

For the best outcome of electrophysiological (EP) diagnostic and/or ablation treatment, a physician may need to determine that each of the multiple electrodes disposed over the spines/arms/preformed-shape-distal-end (these electrodes called hereinafter "functional electrodes") is in physical contact with cavity wall tissue to be diagnosed and/or ablated. For example, when a basket catheter with multiple functional electrodes is used to measure arrhythmia over and/or ablate an ostium of a pulmonary ventricle (PV), typically all of a sub-set of functional electrodes distributed over an entire lateral circumference of the catheter should be positioned so they are in full contact with the PV tissue.

It is not uncommon, however, for some of these functional electrodes to be immersed in blood rather than in contact with tissue. A tissue area under such an electrode will not be characterized correctly, or, in the case of ablation, the applied electrical power in these electrodes may cause unwanted side effects, such as clot formation.

Embodiments of the present invention that are described herein provide a system capable of determining whether a functional electrode is in contact with tissue. The embodiments provide distal-end assemblies comprising (i) at least one functional electrode and (ii) a reference electrode disposed on the assembly or on a distal end of the shaft, such that it does not make contact with tissue. This reference electrode is also called hereinafter "interior electrode" or "center electrode."

In some embodiments, a distal-end assembly of a basket catheter comprises an expandable frame having one or more functional electrodes disposed thereon, and a reference electrode disposed on a backbone of the frame (e.g., on a contraction wire) that is in a volume defined by the frame of the catheter. This backbone-disposed electrode is called hereinafter "interior electrode" or "center electrode."

Using impedance measurements between each functional electrode and the reference electrode, a processor of the EP-mapping/ablation system determines, for each functional electrode, whether or not it is in contact with wall tissue.

In some embodiments, the processor of the system compares impedances measured inside the heart between a functional electrode, which is intended to have contact with cardiac wall tissue of a cardiac chamber, and the interior electrode. When the functional electrode makes tissue contact, the impedances measured are smaller than the impedances measured with the functional electrode in blood by at least a prespecified minimal value. Depending on, for example, the number of electrodes already in full contact with tissue, different minimal values of impedance difference may be prespecified. The prespecified minimal values can be stored, for example, in a look-up table.

The above-mentioned prespecified minimal impedancedifference values are typically determined at an RF frequency of a several tens of kHz, at which cardiac tissue impedance is typically similar or lower than that of blood (both in the range of few 100 Ω or less). Further information on tissue vs blood impedances as a function of RF frequencies is available, for example, in "Medical Instrumentation: Application and Design," Webster (ed.) 3rd Ed., John Wiley & Sons, Inc., New York, 1998.

The disclosed measurement geometry involves comparable path lengths in blood and tissue, so the measured impedances mainly change due to sufficient tissue impedance in parallel to blood impedance. This characteristic of the disclosed technique gives a high degree of certainty to the distinction made by the processor based on the measurements between blood contact and tissue contact.

As a functional electrode approaches a wall tissue, the measured impedance approaches that of an electrode in firm contact with tissue. The sufficient change experienced in impedance between blood and tissue contact, and a sufficient accuracy of measurement, allow, in some embodiments, a proximity estimate of such a functional electrode to wall tissue. Such a measurement gives the change (e.g., drop) due to a relative contribution of tissue impedance to total impedance, which is a continuous function of a distance in blood between the functional electrode and wall tissue.

If physical contact is not achieved for all required functional electrodes, the physician may maneuver the basket catheter to establish more complete contact of the functional electrodes with tissue over the entire lateral perimeter of the basket catheter, and again check the sufficiency and/or existence of contact using the disclosed technique.

In some embodiments, in order to measure a basket catheter position inside the organ, the disclosed system includes a position tracking sub-system that measures impedances between the functional electrodes and surface electrodes. The method, which is further described below, is sometimes called Advanced Catheter Location (ACL). Using a relay assembly, the system can switch electrical connections between the functional electrodes and surface electrodes and between the functional electrodes the interior electrode of the basket catheter in order to interchangeably measure electrode position and degree of functional electrode contact with tissue at the location.

Furthermore, using the relay assembly, the system can switch electrical connections between the functional electrodes and either the reference electrode (for assessing contact) or the surface electrodes (for measuring positions) to a back patch electrode in order to perform unipolar ablation by driving radiofrequency (RF) signals between the functional electrodes and the back patch electrode.

More generally, using the relay assembly, the system can switch electrical connections, under control of the processor, between (i) a first configuration for measuring impedances between the functional electrodes and the reference electrode, and (ii) a second configuration for performing ablation by driving an ablation signal to the functional electrodes.

Typically, the processor is programmed in software containing a particular algorithm that enables the processor to conduct each of the processor-related steps and functions outlined hereinafter.

By determining, in real time, which functional electrode is in contact with tissue and which is not, the disclosed technique may increase the safety and effectiveness of catheterization procedures using multi-electrode basket catheters.

The above technique holds for various types of multi-electrode catheters, such as balloon, multi-arm and lasso catheters, wherever such include at least one referenceelectrode that is immersed in blood pool and cannot contact tissue, such as an electrode disposed on a distal end of a shaft just proximally to the expandable frame of the multi-electrode catheter.

### SYSTEM DESCRIPTION

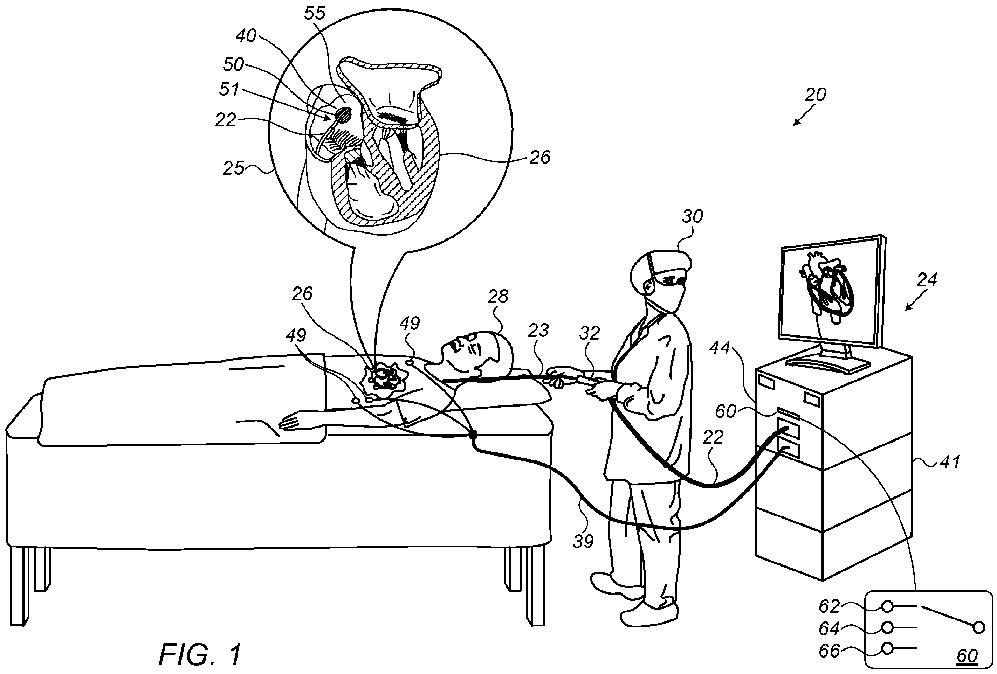

Fig. 1 is a schematic pictorial illustration of a catheter-based electrophysiological (EP) mapping, positiontracking and ablation system 20 comprising a basket catheter 40, in accordance with an embodiment of the present invention. Specifically, basket catheter 40 is used for EP diagnostics and/or therapeutic treatment, such as for identifying and ablating arrhythmogenic cardiac tissue, for example at the left atrium. System 20 is used to determine the position of basket catheter 40, seen in an inset 25 coupled to a distal end of a shaft 22. System 20 is further used to determine, e.g., prior to performing diagnostics and/or ablation, whether each of functional electrodes 50 of basket catheter 40 is in contact with tissue, or immersed in blood pool 55.

Physician 30 navigates basket catheter 40 to a target location in a heart 26 of a patient 28 by manipulating shaft 22 using a manipulator 32 near the proximal end of the catheter and/or deflection from a sheath 23. Basket catheter 40 is inserted, in a collapsed configuration, through sheath 23, and only after the basket is retracted from sheath 23 does basket catheter 40 expand to regain its intended functional shape. By containing basket catheter 40 in a folded configuration, sheath 23 also serves to minimize vascular trauma on its way to the target location.

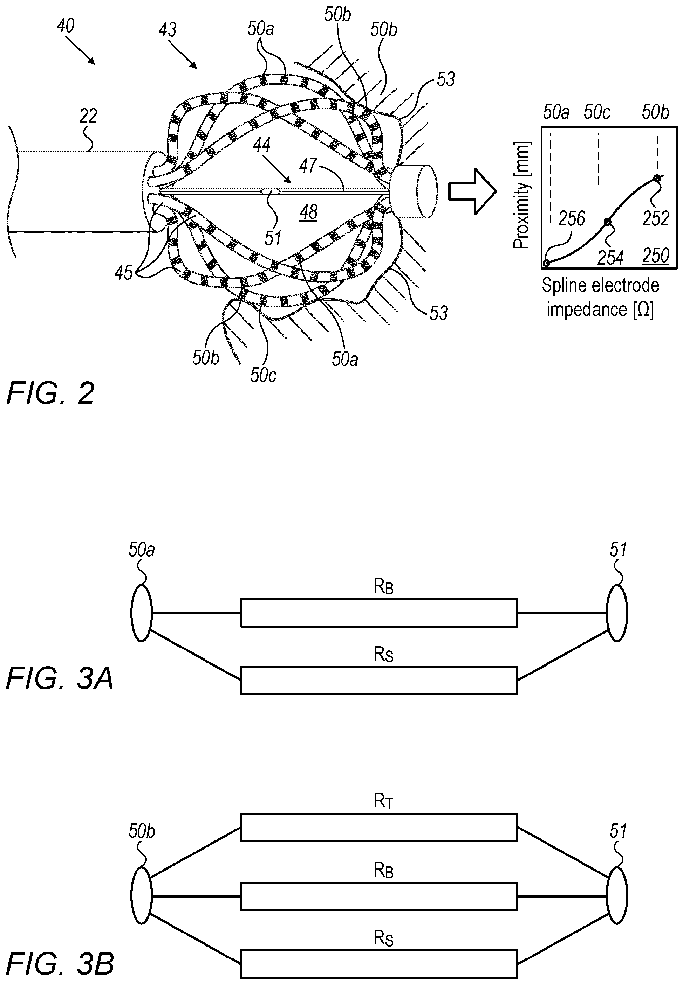

Basket catheter 40 comprises multiple functional electrodes 50, which are disposed on an outer surface of the basket splines. An interior, i.e., center, electrode 51 is disposed on a contraction wire (seen in Fig. 2) inside the expandable frame of the basket. Interior electrode 51 is used to determine whether each of functional electrodes 50 is in contact with tissue or immersed in blood pool 55.

Functional electrodes 50 and interior electrode 51 are connected by wires running through shaft 22 to interface circuits 44 in a console 24. A detailed view of basket catheter 40 with functional electrodes 50 and interior electrode 51 is shown in Fig. 2.

Additionally, using the aforementioned ACL method, functional electrodes 50 can be used to measure a position of basket catheter 40 inside heart 26 by sensing impedances relative to surface electrodes 49, which are seen in the exemplified system as attached by wires running through a cable 39 to the chest of patient 28. The ACL method for tracking the positions of electrodes 50 is implemented in various medical applications, for example in the CARTO^{™} system, produced by Biosense-Webster Inc. (Irvine, California) and is described in detail in U.S. Patents 7,756,576, 7,869,865, 7,848,787, and 8,456,182. Console 24 drives a display 27, which shows the tracked position of basket catheter 40 inside heart 26.

Console 24 comprises a processor 41, typically a generalpurpose computer and a suitable front end and interface circuits 44 for transmitting and receiving signals, such as RF signals and position signals, respectively. Interface circuits 44 may also receive electrocardiograms from surface electrodes 49 and/or from any electrode disposed on the catheter.

In some embodiments, processor 41 controls a relay assembly 60 in system 20 to switch electrical connections between two or more of (i) a first configuration having a connection (62) between the functional electrodes and surface electrodes 49 for measuring impedances between the functional electrodes and one or more body-surface electrodes, (ii) a second configuration having a connection (64) between the functional electrodes and the interior electrode of the basket catheter for measuring impedances between the functional electrodes and the interior electrode, where connections 62 and 64 are used in order to interchangeably measure electrode position and degree of functional electrode contact with tissue at the location, and (iii) a connection (66) between the functional electrodes and a back patch electrode (not shown) in order to perform ablation by driving an RF signal between the functional electrodes and the back patch electrode.

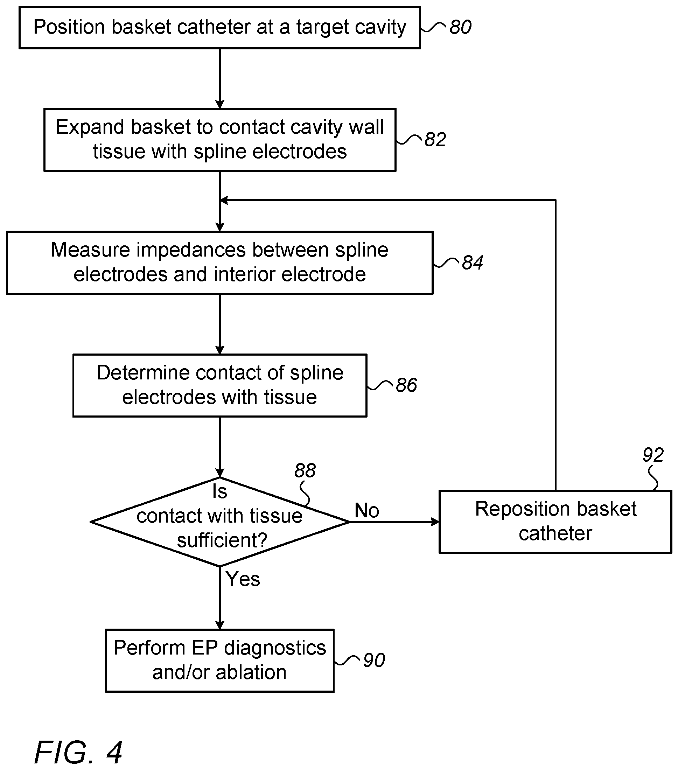

Processor 41 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory. In particular, processor 41 runs a dedicated algorithm as disclosed herein, including in Fig. 4, that enables processor 41 to perform the disclosed steps, as further described below.

Fig. 1 shows only elements related to the disclosed techniques for the sake of simplicity and clarity. System 20 typically comprises additional modules and elements that are not directly related to the disclosed techniques, such as irrigation and temperature modules, and thus are intentionally omitted from Fig. 1 and from the corresponding description.

### MEASURING TISSUE PROXIMITY FOR BASKET CATHETER

Fig. 2 is a perspective, schematic view of basket catheter 40 of Fig. 1 in physical contact with cavity wall tissue, in accordance with an embodiment of the present invention.

The shown portion of catheter 40 comprises a distal end of shaft 22 and a basket-shaped electrode assembly 43 mounted at the distal end. Shaft 22 comprises an elongated tubular construction having a single, axial or center lumen (not shown). The thickness of the outer wall of shaft 22 is not critical, but is preferably thin enough so that the central lumen can accommodate a puller wire, lead wires, sensor cables, as well as other wires, cables or tubes. An example of a catheter body construction suitable for use in connection with the present invention is described and depicted in U.S. Pat. No. 6,064,905.

As shown in Fig. 2, basket-shaped electrode assembly 43 comprises six splines 45 mounted evenly spaced around a backbone 44 comprising contraction wire 47, which is connected to the distal extremity of the electrode assembly 43, and which contracts, retracts and expands the electrode assembly 43 when a tractive or a pushing force is applied longitudinally to contraction wire 47, as the case may be. As seen, the backbone 44 comprising the contraction wire 47 forms a longitudinal axis 62 of symmetry for the electrode assembly 43. Splines 45 are all attached, directly or indirectly, to the contraction wire 47 at their distal ends, and to shaft 22 at their proximal ends. Splines 45 define an inner volume 48 in which center electrode is located and prevented, by the splines, from being near tissue. When the contraction wire 47 is moved longitudinally to expand and contract the electrode assembly 43, the expanded position of the splines 45 are bowed outwardly, and in the contracted position the splines 45 are generally straight. As will be recognized by one skilled in the art, the number of splines 45 can vary as desired, depending on the particular application, so that the electrode assembly 43 preferably has at least three splines and as many as eight splines or more. As used herein, the term "basket shaped" in describing the electrode assembly 43 is not limited to the depicted configuration, but can include other designs, such as spherical or egg-shaped designs that include a plurality of expandable arms connected, directly or indirectly, at their proximal and distal ends.

Each of the functional electrodes 50 on splines 45 is electrically connected to system 20, to an appropriate mapping or monitoring subsystem and/or source of ablation energy by means of an electrode lead wire (not shown). The contraction wire 47 is provided with a center electrode 51, e.g., a cylindrical electrode, the function of which is further described below.

Each of the splines 45 comprise a flexible wire with a non-conductive covering on which one or more ring functional electrodes 50 are mounted. Functional electrodes 50, immersed deep in blood, are denoted 50a, whereas functional electrodes 50 in physical contact with wall tissue 53 (e.g., with endocardial surface 53 of heart 26) are denoted 50b. Electrodes that are in various levels of proximity to wall tissue 53 are denoted 50c. The term "sufficient" can be understood by a graph 250, that shows that an impedance 254 of such electrodes (50c) can be measured to be between an impedance 252 of an electrode 50a and impedance 256 of an electrode 50b. After calibrating graph 250, a processor can estimate, from impedance 254, a proximity of a functional electrode 50 to tissue, e.g., in millimeters. Graph 250 can be saved in a memory of system 20 as a calibrated proximity look-up table. More generally, the processor uses calibrated proximity data that translates between impedance and electrode-tissue distance.

### ESTIMATING PROXIMITY AND/OR CONTACT WITH TISSUE

Figs. 3A and 3B are schematic electrical diagrams of a functional electrode 50 electrically coupled to an interior electrode 51 while the functional electrode is in blood pool 55 and is in contact with wall tissue 53, respectively, in accordance with an embodiment of the present invention. The diagram of Fig. 3A describes a case of functional electrode 50a immersed deep in blood pool 55. As seen, the impedance (e.g., impedance 252) between functional electrode 50a and center electrode 51 equals that of blood, R_{B}, in parallel to a shunt resistance R_{S} that might result from blood and/or tissue and/or other electrically conductive intra-body channel. In brief notation this is represented as |Z_252|=R_{B}||R_{S}.

The diagram of Fig. 3B describes the case of functional electrode 50b completely in contact with wall tissue 53 of Fig. 2. As seen, the impedance between ablation electrode 50b and center electrode 51 is of blood in parallel with tissue, R_{B}∥R_{T}, in parallel to the shunt resistance R_{S}. In brief notation this is represented as |Z_256|=R_{B}∥R_{T}∥R_{S}. As R_{B}∥R_{T}< R_{B}, e.g., by at least few tens of ohms, an amount called hereinafter "impedance threshold", the disclosed method can differentiate between the two cases. Moreover, an in-between value, Z_254, Z_256<Z_254< Z_252 can be measured, and using calibrated graph 250 of Fig.2, a processor may estimate from Z_254 the proximity of electrode 50 to tissue.

The electrical diagrams shown in Figs. 3A and 3B are highly simplified, with the aim of presenting the concept. Actual values may be determined empirically or by a more elaborate electrical model. Figs. 3A and 3B are fully applicable to any electrode, like center electrode 51, that is away from tissue, such as to a proximal electrode disposed on a shaft just proximally to a balloon, a multi-arm, or a lasso catheter, each mounted with multiple tissue sensing/ablation electrodes of similar functionality to that of functional electrodes 50.

Fig. 4 is a flow chart that schematically illustrates a method and algorithm for determining contact of functional electrode 50 with wall tissue 53, in accordance with an embodiment of the present invention. The algorithm according to the present embodiment carries out a process that begins with physician 30 positioning a partially expanded basket catheter 40 at a target location inside a cardiac cavity of heart 26, such as at an ostium of a pulmonary vein, at a basket positioning step 80. Next, physician 30 expands the basket to bring at least a portion (e.g., sub-set) of functional electrodes 50 into full contact with tissue, e.g., over an entire lateral circumference of assembly 43, in a basket expansion step 82. Next, at impedances measurement step 84, system 20 measures impedances between each of functional electrodes 50 and the center (i.e., interior) electrode 51.

At a physical contact determination step 86, based on the measured impedances, processor 41 determines, for each functional electrode 50, whether the electrode is in contact with tissue, as defined above, or at least in sufficient proximity. If, at a contact checking step 88, the processor determines that all functional electrodes 50 are in contact with tissue, the process continues to perform diagnostics and/or ablation, at diagnostics and/or ablation step 90. If, on the other hand, one or more electrodes are determined by processor 41 to be immersed in a blood pool (e.g., blood pool 55 of Fig. 1) and therefore too far away from tissue, physician 30 then repositions basket catheter 40 in an attempt to improve contact, and the process loops back to step 84, to reassess sufficiency of contact.

The example flow chart shown in Fig. 4 is chosen purely for the sake of conceptual clarity. The present embodiment also comprises additional steps of the algorithm, such as acquiring intra-cardiac electrocardiograms, which have been omitted from the disclosure herein purposely in order to provide a more simplified flow chart. In addition, other steps, such as temperature measurements and applying irrigation, are omitted for clarity of presentation.

Although the embodiments described herein mainly address cardiac applications, the methods and systems described herein can also be used in other applications, such as in renal denervation.

It will thus be appreciated that the embodiments described above are cited by way of example, and that the present invention is not limited to what has been particularly shown and described hereinabove. Rather, the scope of the present invention is defined by the claims.

## Claims

1. A system, comprising:
a catheter (40) comprising a distal-end assembly coupled to a distal end of a shaft for insertion into a cavity of an organ of a patient, the distal-end assembly comprising (i) one or more functional electrodes (50) configured to be placed in contact with wall tissue of the cavity and (ii) a reference electrode (51) configured to be placed in the cavity but not in contact with the wall tissue; and
a processor, which is configured to:
estimate one or more impedances between one or more of the functional electrodes and the reference electrode; and
based on the impedances, determine, for at least a functional electrode from among the one or more functional electrodes, whether the functional electrode is in physical contact with the wall tissue, and wherein the catheter is a basket catheter having an expandable frame comprising multiple splines, wherein the functional electrodes are coupled to the splines, and wherein the reference electrode is disposed on a backbone located in an interior of the expandable frame; and
determine that the functional electrode is in physical contact with the wall tissue when the impedances are smaller than impedances measured with the functional electrode in blood by at least a prespecified minimal value, **characterized in that** the prespecified minimal value is different depending on the number of electrodes already in contact with tissue.

2. The system according to claim 1, wherein the processor is further configured to estimate, based on the impedances, for at least a functional electrode from among the one or more functional electrodes, a distance between the functional electrode and the wall tissue.

3. The system according to claim 2, wherein the processor is configured to estimate the distance using calibrated proximity data that translates between impedance and electrode-tissue distance.

4. The system according to claim 1, and comprising a relay assembly that is configured to switch, under control of the processor, between (i) a first configuration for measuring impedances between the functional electrodes and the reference electrode, and (ii) a second configuration for performing a medical procedure using the functional electrodes.

## Patentansprüche

1. System, umfassend:
einen Katheter (40), umfassend eine Distalendanordnung, die an ein distales Ende eines Schafts für eine Einführung in einen Hohlraum eines Organs eines Patienten gekoppelt ist, die Distalendanordnung umfassend (i) eine oder mehrere funktionelle Elektroden (50), die konfiguriert sind, um in Kontakt mit Wandgewebe des Hohlraums platziert zu werden, und (ii) eine Referenzelektrode (51), die konfiguriert ist, um in dem Hohlraum, aber nicht in Kontakt mit dem Wandgewebe platziert zu werden; und
einen Prozessor, der konfiguriert ist zum:
Schätzen einer oder mehrerer Impedanzen zwischen einer oder mehreren der funktionellen Elektroden und der Referenzelektrode; und
basierend auf den Impedanzen, Bestimmen, für mindestens eine funktionelle Elektrode aus der einen oder den mehreren funktionellen Elektroden, ob die funktionelle Elektrode in physischem Kontakt mit dem Wandgewebe steht, und wobei der Katheter ein Korbkatheter ist, der einen expandierbaren Rahmen aufweist, umfassend mehrere Keile, wobei die funktionellen Elektroden an die Keile gekoppelt sind, und wobei die Referenzelektrode auf einem Rückgrat angeordnet ist, das sich in einem Inneren des expandierbaren Rahmens befindet; und
Bestimmen, dass die funktionelle Elektrode in physischem Kontakt mit dem Wandgewebe steht, wenn die Impedanzen um mindestens einen vorgegebenen Minimalwert kleiner sind als Impedanzen, die mit der funktionellen Elektrode in Blut gemessen werden, **dadurch gekennzeichnet, dass** der vorgegebene Minimalwert abhängig von der Anzahl der bereits in Kontakt mit Gewebe stehenden Elektroden unterschiedlich ist.

2. System nach Anspruch 1, wobei der Prozessor ferner konfiguriert ist, um, basierend auf den Impedanzen, für mindestens eine funktionelle Elektrode aus der einen oder den mehreren funktionellen Elektroden einen Abstand zwischen der funktionellen Elektrode und dem Wandgewebe zu schätzen.

3. System nach Anspruch 2, wobei der Prozessor konfiguriert ist, um den Abstand unter Verwendung kalibrierter Näherungsdaten zu schätzen, die zwischen Impedanz und Elektroden-Gewebe-Abstand umrechnen.

4. System nach Anspruch 1, und umfassend eine Relaisanordnung, die konfiguriert ist, um, unter der Steuerung des Prozessors, zwischen (i) einer ersten Konfiguration zum Messen von Impedanzen zwischen den funktionellen Elektroden und der Referenzelektrode und (ii) einer zweiten Konfiguration zum Durchführen eines medizinischen Verfahrens unter Verwendung der funktionellen Elektroden umzuschalten.

## Revendications

1. Système, comprenant :
un cathéter (40) comprenant un ensemble d'extrémité distale couplé à une extrémité distale d'une tige pour une insertion dans une cavité d'un organe d'un patient, l'ensemble d'extrémité distale comprenant (i) une ou plusieurs électrodes fonctionnelles (50) configurées pour être placées en contact avec le tissu de la paroi de la cavité et (ii) une électrode de référence (51) configurée pour être placée dans la cavité mais pas en contact avec le tissu de la paroi ; et
un processeur qui est configuré pour :
estimer une ou plusieurs impédances entre une ou plusieurs des électrodes fonctionnelles et l'électrode de référence ; et
sur la base des impédances, déterminer, pour au moins une électrode fonctionnelle parmi les une ou plusieurs électrodes fonctionnelles, si l'électrode fonctionnelle est en contact physique avec le tissu de la paroi, et dans lequel le cathéter est un cathéter à panier ayant une armature expansible comprenant de multiples branches, dans lequel les électrodes fonctionnelles sont couplées aux branches, et dans lequel l'électrode de référence est disposée sur un axe central situé dans un intérieur de l'armature expansible ; et
déterminer que l'électrode fonctionnelle est en contact physique avec le tissu de la paroi lorsque les impédances sont inférieures aux impédances mesurées avec l'électrode fonctionnelle dans le sang d'au moins une valeur minimale préspécifiée, **caractérisé en ce que** la valeur minimale préspécifiée est différente selon le nombre d'électrodes déjà en contact avec le tissu.

2. Système selon la revendication 1, dans lequel le processeur est en outre configuré pour estimer, sur la base des impédances, pour au moins une électrode fonctionnelle parmi la ou les électrodes fonctionnelles, une distance entre l'électrode fonctionnelle et le tissu de la paroi.

3. Système selon la revendication 2, dans lequel le processeur est configuré pour estimer la distance en utilisant des données de proximité étalonnées qui établissent une correspondance entre l'impédance et la distance électrode-tissu.

4. Système selon la revendication 1, et comprenant un ensemble de relais qui est configuré pour commuter, sous la commande du processeur, entre (i) une première configuration pour mesurer des impédances entre les électrodes fonctionnelles et l'électrode de référence, et (ii) une seconde configuration pour effectuer une procédure médicale en utilisant les électrodes fonctionnelles.
